(19) Europäisches Patentamt
European Patent Office
Office européen des brevets

(11) Veröffentlichungsnummer: **0 148 432**
**B1**

(12) # EUROPÄISCHE PATENTSCHRIFT

(45) Veröffentlichungstag der Patentschrift: **22.07.87**

(51) Int. Cl.⁴: **C 01 B 21/14**, C 07 C 51/42

(21) Anmeldenummer: **84115035.2**

(22) Anmeldetag: **10.12.84**

(54) **Stabilisierte Lösungen von Hydroxylamin oder dessen Salzen in Wasser oder Alkoholen sowie deren Herstellung.**

(30) Priorität: **17.12.83 DE 3345733**

(43) Veröffentlichungstag der Anmeldung: **17.07.85 Patentblatt 85/29**

(45) Bekanntmachung des Hinweises auf die Patenterteilung: **22.07.87 Patentblatt 87/30**

(84) Benannte Vertragsstaaten: **BE CH DE FR GB IT LI NL**

(56) Entgegenhaltungen:
**US-A-3 145 082**
**US-A-3 480 391**
**US-A-3 480 392**
**US-A-3 544 270**

(73) Patentinhaber: **BASF Aktiengesellschaft, Carl-Bosch-Strasse 38, D-6700 Ludwigshafen (DE)**

(72) Erfinder: **Grosskinsky, Otto Alfred, Dr. Chem., Semmelweisstrasse 8, D-6700 Ludwigshafen (DE)**
Erfinder: **Frommer, Elmar, Dr. Chem., Lisztstrasse 117, D-6700 Ludwigshafen (DE)**
Erfinder: **Ritz, Josef, Dr. Chem., Osloer Weg 8, D-6700 Ludwigshafen (DE)**
Erfinder: **Thomas, Erwin, Borngasse 12, D-6713 Freinsheim (DE)**
Erfinder: **Weiss, Franz-Josef, Dr. Chem., Schilfweg 1, D-6706 Neuhofen (DE)**

**EP 0 148 432 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Lösungen von Hydroxylammoniumsalzen zersetzen sich langsam bei Raumtemperatur und schneller bei erhöhter Temperatur. Dies gilt in vermehrtem Maße für Lösungen von freiem Hydroxylamin. Es hat nicht an Versuchen gefehlt, Lösungen von Hydroxylamin und dessen Salzen zu stabilisieren um eine verbesserte Lagerfähigkeit zu erzielen. So verwendet man als Stabilisierungsmittel Harnstoffderivate entsprechend der US-A- 3 544 270. Aus der US-A- 3 480 391 ist bekannt, daß sich Amidoxime als Stabilisatoren eignen. Entsprechend der US-A- 3 480 392 werden hierfür Hydroxamsäuren empfohlen. Aus der US-A- 3 145 082 ist auch schon bekannt, chelatbildende Mittel wie das Natriumsalz der Ethylendiamintetraessigsäure als Stabilisierungsmittel zu verwenden. Die bislang verwendeten Stabilisierungsmittel sind noch verbesserungsbedürftig.

Es war die technische Aufgabe gestellt, stabilisierte Lösungen von Hydroxylamin oder dessen Salzen zur Verfügung zu stellen, die über einen Längeren Zeitraum stabil sind und bei denen insbesondere die Zersetzung von freiem Hydroxylamin minimiert wird, wobei sich die Lösungen beim Lagern nicht verfärben.

Diese Aufgabe wird gelöst durch stabilisierte Lösungen von Hydroxylamin oder dessen Salzen in Wasser oder Alkoholen, gekennzeichnet durch einen Gehalt eines Lactons der Formel

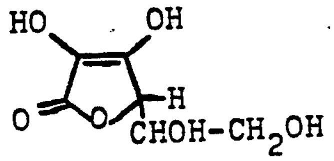

I

Ferner ist ein Gegenstand der Erfindung ein Verfahren zur Herstellung von stabilisierten Lösungen von Hydroxylamin oder dessen Salzen durch Zugabe von Stabilisatoren, dadurch gekennzeichnet, daß man aus der zu stabilisierenden Lösung den darin gelöst enthaltenen molekularen Sauerstoff durch Behandeln mit Stickstoff, der frei von molekularem Sauerstoff ist, entfernt und dann das Lacton der Formel I zusetzt.

Die gemäß der Erfindung stabilisierten Lösungen von Hydroxylamin oder dessen Salzen haben den Vorteil, daß sie über längere Zeit als bisher stabil sind und insbesondere die Zersetzung von freiem Hydroxylamin auf ein Minimum reduziert wird. Die erfindungsgemäß stabilisierten Lösungen haben weiterhin den Vorteil, daß sie sich beim Lagern nicht verfärben.

Erfindungsgemäß geht man von Lösungen von Hydroxylamin oder dessen Salze in Wasser oder Alkoholen z. B. $C_1$- bis $C_4$-Alkanolen aus. Geeignete Salze des Hydroxylamins sind z.B. solche mit starken Mineralsäuren, wie Schwefelsäure, Salpetersäure, Chlorwasserstoffsäure oder solche von Fettsäuren, z.B. Essigsäure oder Propionsäure. Aufgrund der verschiedenen Löslichkeiten liegt Hydroxylamin vorzugsweise gelöst in Wasser oder Alkoholen vor, während dessen Salze vorzugsweise als wäßrige Lösungen vorliegen. Der Gehalt an Hydroxylamin oder dessen Salzen beträgt in der Regel von 10 bis 70 Gew.-%. Besonders bevorzugt geht man von Lösungen von Hydroxylamin in Wasser aus. Solche Ausgangslösungen haben in der Regel einen pH-Wert von 8 bis 11.

Als Stabilisator verwendet man erfindungsgemäß ein Lacton der Formel

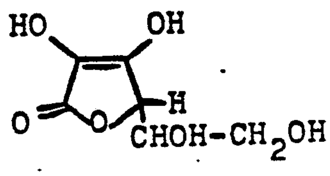

I

Das Lacton der Formel I ist bekannt aus Beilstein E III/VI 1B, 3038-3047. Vorteilhaft verwendet man das Lacton der Formel I in Mengen von 0,005 bis 1 Gew.-%, insbesondere von 0,01 bis 0,1 Gew.-%, bezogen auf die zu stabilisierende Lösung an.

Stabilisierte Lösungen von Hydroxylamin oder dessen Salzen in Wasser oder Alkoholen werden erfindungsgemäß hergestellt indem man aus den zu stabilisierenden Lösungen zunächst den darin gelöst enthaltenen molekularen Sauerstoff durch Behandeln mit Stickstoff, der frei von molekularem Sauerstoff ist, verdrängt. Dies erzielt man beispielsweise dadurch, daß man durch die zu stabilisierende Lösung sauerstoffreien Stickstoff leitet, z.B. für 5 bis 10 Minuten. Der angewandte Stickstoff enthält vorteilhaft weniger als 2 ppm molekularen Sauerstoff. Dann gibt man das Lacton der Formel I zu und löst dieses in der zu stabilisierenden Lösung auf. Hierbei hält man vorteilhaft eine Temperatur von 5 bis 40°C ein. Es ist auch möglich, die Stabilisatoren bereits in gelöstem Zustand z.B. gelöst in Wasser oder in $C_1$- bis $C_4$-Alkanolen den zu stablisierenden Lösungen zuzugeben.

Es versteht sich, daß es von Vorteil ist, eine Kontamination von zu stabilisierenden Lösungen mit Schwermetallen, insbesondere Kupfer oder Edelmetallen zu vermeiden. Vorteilhaft soll der Gehalt an solchen Schwermetallen kleiner 1 ppm betragen. Ferner ist es von Vorteil, energiereiche Strahlung durch geeignet eingefärbte Glasbehälter auszuschalten. Schließlich ist es von Vorteil, die stabilisierten Lösungen bei Temperaturen von $<40°C$, z.B. bei Temperaturen von 5 bis 20°C zu lagern.

Stabilisierte Lösungen von Hydroxylamin oder dessen Salze eignen sich zur Herstellung von Oximen.

Der erfindungsgemäße Gegenstand sei an folgendem Beispiel veranschaulicht

**Beispiel**

Durch eine wäßrige Lösung von Hydroxylamin wird bei 20°C 10 Minuten sauerstoffreier Stickstoff geleitet und dann der Stabilisator zugefügt. Die Konzentration an Hydroxylamin, die zugegebene Menge und Art des Stabilisators sowie die in Abhängigkeit von der Zeit erzielten Ergebnisse sind aus folgender Tabelle zu entnehmen.

**Tabelle**

| Stabilisator | °C | | | | |
|---|---|---|---|---|---|
| 2,3-Dihydrohexono- -1,4-lacton | 25 | 0 | 293 | 462 | Stunden |
| 25 mg/mol $NH_2OH$ | | 540,15 | 539,98 | 535,63 | g/l $NH_2OH$ |

**Patentansprüche**

1. Stabilisierte Lösungen von Hydroxylamin oder dessen Salzen in Wasser oder Alkoholen, gekennzeichnet durch einen Gehalt eines Lactons der Formel

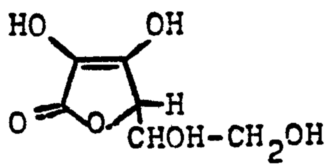

I

2. Stabilisierte Lösungen nach Anspruch 1, gekennzeichnet durch einen Gehalt von 0,005 bis 1 Gew.-% eines Lactons der Formel 1, bezogen auf die zu stabilisierende Lösung.

3. Stabilisierte Lösungen nach den Ansprüchen 1 und 2, dadurch gekennzeichnet, daß man von einer wäßrigen Lösung mit einem Gehalt von 10 bis 70 Gew.-% Hydroxylamin ausgeht.

4. Stabilisierte Lösungen nach den Ansprüchen 1 bis 3, gekennzeichnet durch einen Gehalt von 0,01 bis 0,1 Gew.-% an Lacton der Formel I.

5. Verfahren zur Herstellung von stabilisierten Hydroxylaminlösungen oder dessen Salzen in Wasser oder Alkoholen durch Zusatz von Stabilisatoren, dadurch gekennzeichnet, daß man aus den zu stabilisierenden Lösungen den darin gelöst enthaltenen molekularen Sauerstoff durch Behandeln mit Stickstoff, der frei von molekularem Sauerstoff ist, entfernt und dann das Lacton der Formel I zusetzt.

**Claims**

1. A stabilized solution of hydroxylamine or its salts in water or an alcohol, which contains a lactone of the formula

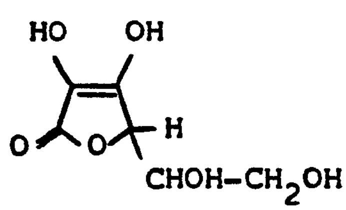

I.

2. A stabilized solution as claimed in claim 1, which contains from 0.005 to 1% by weight, based on the solution to be stabilized, of a lactone of the formula I.

3. A stabilized solution as claimed in claims 1 and 2, wherein an aqueous solution containing from 10 to 70% by weight of hydroxylamine is used as starting material.

4. A stabilized solution as claimed in claims 1 to 3, which contains from 0.01 to 0.1% by weight of a lactone of the formula I.

5. A process for the preparation of a stabilized solution of hydroxylamine or its salts in water or an alcohol by adding a stabilizer, wherein the molecular oxygen dissolved in the solution to be stabilized is removed from

this solution by treatment with nitrogen which is free of molecular oxygen, and a lactone of the formula I is then added.

## Revendications

1. Solutions aqueuses ou alcooliques d'hydroxylamine ou de sels de celle-ci, stabilisées par une lactone de la formule

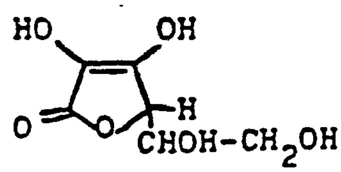

I.

2. Solutions stabilisées suivant la revendication 1, caractérisées en ce qu'elles contiennent entre 0,005 et 1 % en poids par rapport à la solution à stabiliser d' une lactone de la formule I.

3. Solutions stabilisées suivant l'une des revendications 1 et 2, caractérisées en ce que la solution de départ est une solution aqueuse d'une teneur en hydroxylamine comprise entre 10 et 70 % en poids.

4. Solutions stabilisées suivant l'une des revendications 1 à 3, caractérisées par une teneur en lactone de la formule I de 0,01 à 0,1 % en poids.

5. Procédé de préparation de solutions aqueuses ou alcooliques d'hydroxyl-amine ou de sels de celle-ci, stabilisées par l'addition d'un agent stabilisant, caractérisé en ce que l'on élimine d'abord des solutions à stabiliser l'oxygène moléculaire qui y est dissous à l'aide d'azote exempt d'oxygène moléculaire, puis on y ajoute la lactone de la formule I.